(19) Europäisches Patentamt
European Patent Office
Office européen des brevets

(11) **EP 2 199 276 B1**

(12) **EUROPÄISCHE PATENTSCHRIFT**

(45) Veröffentlichungstag und Bekanntmachung des Hinweises auf die Patenterteilung:
**22.07.2015 Patentblatt 2015/30**

(51) Int Cl.:
*C07C 263/10* (2006.01)    *C07C 265/12* (2006.01)

(21) Anmeldenummer: **09015100.2**

(22) Anmeldetag: **05.12.2009**

(54) **Verfahren zur Herstellung von Isocyanaten in der Gasphase**

Method for manufacturing isocyanates in the gaseous phase

Procédé destiné à la fabrication d'isocyanates en phase gazeuse

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **19.12.2008 DE 102008063991**

(43) Veröffentlichungstag der Anmeldung:
**23.06.2010 Patentblatt 2010/25**

(73) Patentinhaber: **Bayer Intellectual Property GmbH**
**40789 Monheim (DE)**

(72) Erfinder:
• **Biskup, Klaus, Dr.**
 **25712 Buchholz (Dithm.) (DE)**
• **Bruns, Rainer, Dr.**
 **51373 Leverkusen (DE)**
• **Lorenz, Wolfgang, Dr.**
 **41540 Dormagen (DE)**
• **Padeken, Lars, Dr.**
 **25524 Itzehoe (DE)**
• **Pennemann, Bernd, Dr.**
 **51467 Bergisch Gladbach (DE)**
• **Pohl, Fritz, Dr.**
 **25541 Brunsbüttel (DE)**
• **Rausch, Andreas, Dr.**
 **41464 Neuss (DE)**
• **Steffens, Friedhelm**
 **51373 Leverkusen (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**EP-A1- 0 570 799    EP-A1- 1 449 826**
**EP-A1- 1 935 876    US-A- 5 391 683**

**Beschreibung**

Die Erfindung betrifft ein Verfahren zur Herstellung von meta-Toluylendiisocyanat durch Phosgenierung von meta-Toluylendiamin in der Gasphase.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen. Eine Möglichkeit der Herstellung von Isocyanaten ist die Umsetzung der Amine mit dem Phosgen in der Gasphase. Diese üblicherweise als Gasphasenphosgenierung bezeichnete Verfahrensführung zeichnet sich dadurch aus, dass die Reaktionsbedingungen so gewählt werden, dass zumindest die Reaktionskomponenten Amin, Isocyanat und Phosgen, bevorzugt jedoch sämtliche Edukte, Produkte und Reaktionszwischenprodukte, bei den gewählten Bedingungen gasförmig sind. Vorteile der Gasphasenphosgenierung sind unter anderem ein verringerter Phosgen-Hold-Up, die Vermeidung schwer phosgenierbarer Zwischenprodukte und erhöhte Reaktionsausbeuten. Die vorliegende Erfindung betrifft ausschließlich die Gasphasenphosgenierung.

Es sind aus dem Stand der Technik verschiedene Verfahren zur Herstellung von Diisocyanaten durch Umsetzung von Diaminen mit Phosgen in der Gasphase bekannt.

Speziell die Phosgenierung von aliphatischen Diaminen in der Gasphase wurde bereits häufig beschrieben. So offenbart EP 289 840 B1 ein Verfahren zur Herstellung von Diisocyanaten durch Phosgenierung der entsprechenden Diamine in der Gasphase, dadurch gekennzeichnet, dass man die dampfförmigen Diamine, gegebenenfalls verdünnt mit einem Inertgas oder mit den Dämpfen eines inerten Lösungsmittels, und Phosgen getrennt auf Temperaturen von 200°C bis 600°C erhitzt und kontinuierlich in einem zylindrischen Reaktionsraum unter Aufrechterhaltung einer turbulenten Strömung miteinander zur Reaktion bringt. Das den Reaktionsraum verlassende Gasgemisch wird durch ein inertes Lösungsmittel geleitet, welches bei einer Temperatur oberhalb der Zersetzungstemperatur des dem Diamin entsprechenden Carbamidsäurechlorids gehalten wird. Das sich dabei in dem inerten Lösungsmittel lösende Diisocyanat wird einer destillativen Aufarbeitung unterzogen.

Auch die Umsetzung aromatischer Diamine mit Phosgen in der Gasphase zu den entsprechenden Diisocyanaten ist in der Literatur beschrieben.

EP 593 334 B1 offenbart ein Verfahren zur Herstellung von aromatischen Diisocyanaten in der Gasphase, wobei ein Rohrreaktor verwendet wird. Darin wird eine Vermischung der Edukte durch eine Einengung der Wände erreicht. Die Umsetzung erfolgt im Temperaturbereich von 250 bis 500°C. Das Verfahren ist jedoch problematisch, da die Vermischung der Eduktströme allein über eine Verengung der Rohrwand im Vergleich zur Anwendung eines richtigen Mischorgans schlecht funktioniert. Eine schlechte Vermischung führt üblicherweise zu einer unerwünscht hohen Feststoffbildung.

Es hat nicht an Versuchen gefehlt, diese insbesondere bei der Umsetzung von aromatischen Diaminen mit Phosgen in der Gasphase zu beobachtende Bildung von Feststoffen zu minimieren um so eine großtechnisch durchführbare Phosgenierung von aromatischen Diaminen in der Gasphase zu ermöglichen. Dabei fokussieren sich die Verbesserungen in dem Verfahren zur großtechnischen Phosgenierung von aromatischen Aminen in der Gasphase auf die Verbesserung der Vermischung der Eduktströme sowie eine Vergleichmäßigung der Strömung in dem Gasphasenreaktor, die zu einer verlängerten Standzeit des Gasphasenreaktors führen.

EP 570 799 B1 offenbart ein Verfahren zur Herstellung von aromatischen Diisocyanaten, dadurch gekennzeichnet, dass die Umsetzung des zugehörigen Diamins mit dem Phosgen in einem Rohrreaktor oberhalb der Siedetemperatur des Diamins innerhalb einer mittleren Verweilzeit von 0,5 bis 5 Sekunden durchgeführt wird, und bei dem die mittlere Abweichung von der mittleren Verweilzeit weniger als 6 % beträgt. Nach der Lehre von EP 570 799 B1 führen sowohl zu lange als auch zu kurze Verweilzeiten zu unerwünschter Feststoffbildung, so dass eine Vergleichmäßigung der Strömung in dem Reaktionsraum erforderlich und vor allem eine Rückvermischung der Komponenten in dem Reaktionsraum auszuschließen ist.

Maßnahmen zur Vergleichmäßigung der Strömungsverhältnisse sind ebenfalls Gegenstand von EP 1 362 847 B1. EP 1 362 847 B1 offenbart ein Verfahren zur Herstellung von aromatischen Diisocyanaten in der Gasphase, bei dem durch eine verbesserte Reaktionsführung in Rohrreaktoren durch strömungstechnische Maßnahmen wie der Vergleichmäßigung und Zentrierung der Eduktströme zeitliche Schwankungen der Temperatur und eine Asymmetrie in der Temperaturverteilung vermieden werden, die nach der Lehre von EP 1 362 847 B1 zu Anbackungen und Verstopfungen im Reaktor und damit zur Verkürzung der Standzeit der Reaktoren führen.

Nach der Lehre von EP 1 449 826 A1 steht bei der Umsetzung der aromatischen Diamine mit Phosgen in der Gasphase die Umsetzung des Phosgens mit dem Diamin zu dem Diisocyanat in Konkurrenz zu der Folgereaktion des Diamins mit dem Diisocyanat zum entsprechenden Harnstoffoligomer, wobei eine verbesserte Mischung der Edukte Phosgen und Diamin bei gleichzeitiger Vermeidung von Rückströmung im Rohrreaktor die Selektivität der Diisocyanatbildung erhöht und die Harnstoffbildung reduziert. Dadurch lassen sich nach der Lehre von EP 1 449 826 A1 die Mengen an Kondensationsprodukt im Rohrreaktor verringern, die aufgrund ihrer Ablagerung an der Reaktorwand zu einer Verkleinerung des freien Rohrquerschnitts und zu allmählichem Druckanstieg im Reaktor führen und letztlich die Standzeit des Verfahrens bestimmen. Apparative Lösungen zur verbesserten Mischung der Edukte offenbaren ebenfalls EP 1 526 129 A1, DE 103 59 627 A1 und WO 2007/028 715 A, wobei strömungstechnische Maßnahmen zur Drallerzeugung (EP 1 526 129 A1), konzentrisch angeordnete Ringspaltdüsen mit singulärer (DE 103 59 627 A1) oder mehrfacher Amineinspeisung (WO 2007/028 715 A) wie auch mehrere, parallel zur Achse eines Rohrreaktors angeordnete Amindüsen (EP 1 449 826 A1) zum Einsatz kommen.

Doch nicht nur die physikalisch-technischen Reaktionsbedingungen sondern ebenfalls die Eigenschaften der in die Umsetzung mit Phosgen in der Gasphase eingesetzten aromatischen Diamine waren Gegenstand offenbarter Verfahren.

So müssen nach der Lehre von WO 2008 / 071 564 A Amine, die in einer Gasphasenphosgenierung zu den entsprechenden Isocyanaten umgesetzt werden sollen, bestimmte Erfordernisse erfüllen. Danach sind speziell solche Amine geeignet, die sich während der Dauer der Reaktion unter den herrschenden Reaktionsbedingungen in dem Gasphasenreaktor zu höchstens 2 mol%, besonders bevorzugt zu höchstens 1 mol% und ganz besonders bevorzugt zu höchstens 0,5 mol% zersetzen. Nach der Lehre von WO 2008 / 071 564 A sind dies aliphatische oder cyclische Amine. Gemäß WO 2008 / 071 564 A können auch aromatische Amine verwendet werden, sofern sich diese ohne signifikante Zersetzung in die Gasphase überführen lassen. Als bevorzugt geeignete aromatische Amine offenbart WO 2008 / 071 564 A Toluylendiamin (TDA), als 2,4-oder 2,6-Isomer oder als Gemisch davon, beispielsweise als 80:20 bis 65:35 (mol/mol) Gemisch, Diaminobenzol, 2,6-Xylidin, Naphthyldiamin und 2,4'-oder 4,4'-Methylen(diphenylamin) (MDA) oder Isomerengemische davon. Anweisungen, wie sich die als bevorzugt geeignet bezeichneten aromatischen Diamine ohne signifikante Zersetzung in die Gasphase überführen lassen, sind der WO 2008 / 071 564 A jedoch nicht zu entnehmen.

Den Einsatz aromatischer Amine, die bevorzugt ohne Zersetzung in die Gasphase überführt werden können, empfiehlt auch EP 1 935 876 A1. Diese Schrift offenbart ein Verfahren zur Herstellung von Isocyanaten in der Gasphase, bei dem der Reaktionsraum weder Heizflächen, die Anlass zu einer thermischen Belastung mit der Folge von Folgereaktionen wie Isocyanurat- oder Carbodiimidbildung geben können, noch Kühlflächen, die Anlass einer Kondensation mit der Folge von Ablagerungen geben können, aufweist.

Eine spezielle Verdampfungstechnik unter Beachtung der thermischen Belastung der in eine Gasphasenphosgenierung eingesetzten Amine offenbart EP 1 754 698 A1. Nach der Lehre von EP 1 754 698 A1 werden die in dem Reaktor zur Umsetzung der eingesetzten Amine mit Phosgen zu beobachtenden Ablagerungen zum Ersten durch die Zersetzung der eingesetzten Amine während der Reaktion verursacht. Zum Zweiten führen lange Verweilzeiten in der Verdampfung und Überhitzung speziell bei Einsatz von aliphatischen Aminen zu einer partiellen Zersetzung der eingesetzten Amine unter Ammoniak-Abspaltung. Diese speziell bei Einsatz von aliphatischen Aminen zu beobachtende partielle Zersetzung unter Ammoniak-Abspaltung während der Verdampfung vermindert nicht nur die Ausbeute, sondern es bilden sich bei der nachfolgenden Phosgenierungsreaktion auch Ablagerungen von Ammoniumchlorid in den nach geschalteten Rohrleitungen und Apparaten. Die Anlagen müssen dann relativ häufig gereinigt werden, wobei entsprechende Produktionsverluste entstehen. EP 1 754 698 A1 beschreibt, dass diese Nachteile insbesondere bei den üblicherweise für die Verdampfung und Überhitzung der Amine eingesetzten Rohrbündelwärmetauschern, Plattenwärmetauschern oder Fallfilmverdampfern auftreten. Als technische Lösung offenbart diese Schrift die Ammoniak-Abspaltung während der Verdampfung dadurch zu unterdrücken, dass für die Verdampfung und Überhitzung der aliphatischen Amine spezielle Milli- oder Mikrowärmetauscher eingesetzt werden sollten. In dem offenbarten Verfahren werden die Amine in dem Verdampfer vollständig verdampft, so dass bewusst auf Kreislaufströmen durch die Apparate verzichtet wird, so dass die Apparate nur einmal von dem Amin durchströmt werden.

Nachteilig an der in der Schrift EP 1 754 698 A1 offenbarten Mikrowärmetauschern sind die sehr kleinen Kanäle, so dass bereits sehr kleine Feststoffmengen, die in technischen Prozessen stets vorhanden sind, zu einer Verstopfung führen, und somit die Standzeit der Verdampfers reduzieren. Weiterhin nachteilig an der offenbarten Totalverdampfung der Amine ist, dass die Amine keine nicht verdampfbaren Bestandteile enthalten dürfen, weil sich diese sonst zwangsläufig als fester Rückstand auf der Verdampferoberfläche ablagern, damit den Wärmeübergang verschlechtern und schließlich zur Verstopfung des Verdampfers führen. Die Bereitstellung von Aminen in der geforderten Qualität ist im technischen Prozess jedoch sehr aufwendig und teuer. Somit wird durch die Lehre dieser Schrift zwar die Standzeit des Reaktors verbessert, die Standzeit des Verdampfersystems jedoch deutlich verschlechtert, so dass die gesamte Standzeit der Produktionsanlage nicht vorteilhaft verbessert wird.

Die Minimierung der thermischen Belastung der Amine bei ihrer Verdampfung zur Umsetzung mit Phosgen in der Gasphase ist ebenfalls Gegenstand von EP 1 935 876 A1. EP 1 935 876 A1 offenbart, dass die Amine vor der Umsetzung mit Phosgen in der Regel verdampft und auf 200°C bis 600°C erhitzt und gegebenenfalls verdünnt mit einem Inertgas wie $N_2$, He, Ar oder mit den Dämpfen eines inerten Lösungsmittels, z.B. aromatischen Kohlenwasserstoffen ggf. mit Halogensubstitution, wie z.B. Chlorbenzol oder o-Dichlorbenzol, dem Reaktionsraum zugeführt werden. Nach Lehre dieser Schrift kann die Verdampfung der Ausgangsamine in allen bekannten Verdampfungsapparaturen erfolgen, bevorzugt werden Verdampfungssysteme eingesetzt, bei denen ein kleiner Arbeitsinhalt mit einer hohen Umwälzleistung über einen Fallfilmverdampfer geführt wird und dabei zur Minimierung der thermischen Belastung der Ausgangsamine der Verdampfungsvorgang - wie oben ausgeführt - ggf. unter Zuspeisung von Inertgas und / oder Dämpfen eines inerten Lösungsmittels unterstützt wird.

Trotz der Versuche, die Umsetzung von aromatischen Aminen mit Phosgen in der Gasphase zu optimieren und dabei insbesondere die bei der Umsetzung von aromatischen Diaminen mit Phosgen in der Gasphase zu beobachtende Bildung von Feststoffen zu minimieren, besteht ein weiterer Bedarf zur Verbesserung der Gasphasenphosgenierung aromatischer Diamine, um so eine großtechnisch durchführbare Phosgenierung von aromatischen Diaminen in der Gasphase zu ermöglichen. Überraschenderweise wurde nun gefunden, dass die Gasphasenphosgenierung aromatischer Diamine im großtechnischen Maßstab auch von der Qualität der aromatischen Diamine abhängt und dass durch die Kombination der Qualität der eingesetzten Amine mit einer speziellen Verdampfungstechnologie die Standzeiten von Anlagen zur Gasphasenphosgenierung wesentlich verbessert werden.

Die erfindungsgemäße Verbesserung des Verfahrens zur Gasphasenphosgenierung aromatischer Amine erfordert das Verständnis der Zusammensetzung der eingesetzten Rohstoffe.

Es ist bekannt, dass aromatische Amine durch katalytische Hydrierung von entsprechenden aromatischen Dinitroverbindungen hergestellt werden können. Die Hydrierung kann unter Mitverwendung von Lösungsmitteln wie Methanol, Ethanol oder Isopropanol aber auch ohne solche Fremdlösungsmittel erfolgen. Die Hydrierung kann mit Hilfe von im Reaktionsgemisch dispergierten Katalysatoren durchgeführt werden, die dann durch Sedimentation oder Filtration abgetrennt und gegebenenfalls in den Prozess zurückgeführt werden, beziehungsweise durch Membranfiltration im Reaktionssystem zurückgehalten werden. Als Katalysator können dotierte oder undotierte Raney-Nickel-Katalysatoren oder auch Metallkatalysatoren, die beispielsweise Iridium, Kobalt, Kupfer, Palladium oder Platin enthalten, verwendet werden. Derartige Verfahren sind aus dem Stand der Technik bekannt, z.B. aus DE 2 135 154 B, DE 3 734 344 A1, EP 634 391 B1, DE 4 435 839 A1, EP 1 287 884 B1, EP 978 505 B1, EP 1 033 361 B1. Bei der Hydrierung entstehen neben dem aromatischen Amin und dem Zwangskoppelprodukt Wasser auch organische Nebenprodukte. Die organischen Nebenprodukte werden üblicherweise entweder nach der Lage ihres Siedepunkts relativ zum aromatischen Amin in die Gruppe der Leichtsieder und Hochsieder oder nach chemischen Gesichtspunkten in verschiedene Gruppen aufgeteilt.

Im Falle der technischen Nitrierung von Toluol zu Dinitrotoluol (DNT) und dessen Hydrierung zu Toluylendiamin (TDA) wird als Produkt ein TDA-Rohgemisch erhalten, das neben dem Zwangskoppelprodukt Wasser und Toluylendiamin mehrere solcher organischer Nebenprodukte enthält. Der nicht-wässrige Anteil des TDA-Rohgemisches besteht im Wesentlichen aus 92 bis 96 Gew.-% der m-TDA-Isomeren (2,4- und 2,6-TDA), aus weniger als 1 Gew.% des para-TDA-Isomeren (2,5-TDA), 3 bis 5 Gew.-% Leichtsieder und 0,2 bis 2 Gew.-% Hochsieder, wobei sich die Prozentsätze jeweils zu 100 Gew.-% ergänzen.

**m-TDA-Isomere** im Sinne der vorliegenden Erfindung bedeutet dabei ein Isomerengemisch aus 2,4-TDA und 2,6-TDA. Bevorzugt wird ein Isomerengemisch von 78 bis 82 Gew.-% 2,4-TDA und 18 bis 22 Gew.-% 2,6-TDA erreicht. In das erfindungsgemäße Verfahren zur Umsetzung aromatischer Diamine mit Phosgen in der Gasphase sind jedoch ebenfalls m-TDA-Isomerengemische mit hiervon abweichenden Isomerenverhältnissen wie auch der getrennte Einsatz der technisch reinen 2,4- oder 2,6-TDA-Isomere möglich. Diesem wird durch die Verwendung des Begriffs meta-Toluylendiamin an geeigneter Stelle Rechnung getragen.

Im Sinne der vorliegenden Erfindung sind **Leichtsieder** Verbindungen, die einen niedrigeren Siedepunkt als beide m-TDA-Isomere haben und **Hochsieder** solche Verbindungen, die einen höheren Siedepunkt als beide m-TDA-Isomere haben.

Unter chemischen Gesichtspunkten besteht der nicht-wässrige Anteil des TDA-Rohgemisches neben den TDA-Isomeren aus Toluidinen und Ammoniak, die jeweils den Leichtsiedern zuzurechnen sind, sowie aus cycloaliphatischen Aminen. Zur Gruppe der **cycloaliphatischen Amine** gehören im Sinne dieser Erfindung Verbindungen, die durch Hydrierung des aromatischen Rings aus einem der TDA-Isomeren oder aus Toluidin gebildet wurden und gegebenenfalls sauerstoffhaltige Gruppen wie Keto- oder Hydroxygruppen enthalten können. Cycloaliphatische Amine können Leicht- oder Hochsieder sein.

Die Zusammensetzung von TDA-Isomerengemischen und ihr Gehalt an Leichtsiedern und cycloaliphatischen Aminen werden üblicherweise mit gaschromatographischen Methoden ermittelt, die dem Fachmann experimentell leicht zugänglich sind. Beispielsweise ist die Methode von Willeboordse et al. dazu geeignet (Willeboordse, F.; Quick, Q.; Bishop, E.T. "Direct gas chromatographic analysis of isomeric diaminotoluenes" Analytical Chemistry 1968, 40 (10), 1455-1458).

Durch die oxidative Kupplung zweier TDA-Isomeren bilden sich oft farbige, oligomere Spezies. Diese Spezies sind der Gruppe der Hochsieder zuzurechnen und werden im Allgemeinen als **TDA-Rückstand** bezeichnet (Krauter, J.G.E.; Groß, M.; Panster, P.: "Influence of Hydrogen Supply on By-Product Formation during the Hydrogenation of DNT to TDA", Science and Technology in Catalysis 2002, 427-430). WO 2005/066113 A1 beschreibt den TDA-Rückstand als Oligomere und Polymere, im Wesentlichen bestehend aus Azo-, Azoxy- oder Hydrazinverbindungen. Zusätzlich weist diese Schrift darauf hin, dass der TDA-Rückstand auch Reste des Katalysators, also Schwermetalle wie Iridium, Kupfer, Kobalt, Nickel, Eisen, Palladium oder Platin enthalten kann. EP 659 173 B1 beschreibt als mögliche Bestandteile des TDA-Rückstandes Diphenylmethane, Diphenylamine, Acridine und Phenazine, also Verbindungen, die mindestens zwei aromatische Ringe aufweisen.

Dem Stand der Technik Rechnung tragend, fasst in der vorliegenden Erfindung der Begriff TDA-Rückstand ebenfalls organische Verbindungen zusammen, die mindestens zwei aromatische Ringe aufweisen aber unterschiedlichste funktionelle Gruppen tragen können. Weiterhin ist im Sinne der vorliegenden Erfindung zu verstehen, dass TDA-Rückstand ein Gemisch der genannten organischen Verbindungen mit Resten des Katalysators, also Schwermetallen wie z.B. Iridium, Kupfer, Kobalt, Nickel, Eisen, Palladium und oder Platin darstellen kann.

Der Gehalt an TDA-Rückstand wird üblicherweise durch Rückstandsdestillation ermittelt, wobei der Massenanteil des TDA-Rückstandes in einer Probe durch Wägung ermittelt wird, bevor und nachdem TDA-Isomere, cycloaliphatische Amine und gegebenenfalls weitere Leichtsieder abdestilliert wurden. Der Gehalt an **Schwermetallen,** dies sind alle Metalle die im Periodensystem der Elemente eine höhere Ordnungszahl aufweisen als das Element Titan, kann durch dem Fachmann bekannte Methoden mittels Atomadsorptionsspektroskopie ermittelt werden.

Das in der Hydrierung erhaltene TDA-Rohgemisch wird üblicherweise zur weiteren Verwendung gereinigt. Die Reinigung kann dabei durch Destillation, Kristallisation, und / oder thermische Nachbehandlung sowie chemische Oxidations- oder Reduktionsprozesse erfolgen.

In großtechnischen Verfahren wird der Reinigungsprozess bevorzugt destillativ durchgeführt, und so das Reaktionswasser sowie Leichtsieder wie Ammoniak, Hexahydro-Toluidin, Hexahydro-Toluylendiamin und gegebenenfalls Lösemittel teilweise oder vollständig entfernt. Diese Wasser-, Leichtsieder- und gegebenenfalls auch Lösemittel-Abtrennung kann ein- oder mehrstufig erfolgen. Bevorzugt wird daran anschließend eine destillative Entfernung eines oder mehrerer der ortho-Toluylendiamin-Isomere durchgeführt, wobei diese destillative o-TDA-Abtrennung ein- oder mehrstufig erfolgen kann. Dabei wird der Gehalt an o-TDA bevorzugt auf weniger als 0,3 Gew.-% reduziert (Kirk-Othmer Encyclopedia of Chemical Technology, A.R. Cartolano: Toluenediamine, John Wiley & Sons, 2001).

Nach den Destillationsschritten zur Aufarbeitung des TDA-Rohgemisches kann eine weitere Aufkonzentrierung des m-TDAs dadurch erfolgen, dass der TDA-Rückstand aus dem verbliebenen m-TDA abgetrennt wird. Nach dem Stand der Technik erfolgt die Abtrennung des TDA-Rückstands aus dem m-TDA üblicherweise wiederum durch den Einsatz destillativer Methoden. Es mangelte nicht an Versuchen den TDA-Rückstand kontinuierlich und unter möglichst geringem Energieeinsatz und Verlust an m-TDA abzutrennen. All diese Versuche führen zu einem m-TDA, das im technischen Sinne als frei von TDA-Rückstand bezeichnet werden kann, aber auch ein solches im technischen Sinne reines m-TDA ist nie vollständig rückstandsfrei, da sich gewisse Rückstandsmengen bei thermischer Belastung erneut bilden.

EP 659 173 B1 nennt die Abtrennung von TDA-Rückstand aus m-TDA als vorteilhaft, da dadurch die Bildung von höhermolekularen Verbindungen bei der Umsetzung des Amins mit Phosgen zu dem Diisocyanat in der flüssigen Phase verringert wird und damit die erforderliche Austragung dieser höhermolekularen Komponenten aus dem TDI-Prozess in geringerem Maße stattfinden muss (Ullmann, 4. Auflage, Band 13, S.351). Weiterhin behauptet EP 659 173 B1, dass nur rückstandsfreies m-TDA in der Gasphasenphosgenierung einsetzbar ist. Allerdings wird diese Behauptung, dass ausschließlich rückstandsfreies TDA für die Gasphasenphosgenierung verwendet werden kann in der Schrift EP 659 173 B1 nicht weiter ausgeführt.

Insgesamt offenbart EP 659 173 B1 ein Verfahren zur Rückstandsabtrennung, in dem unter Zuhilfenahme von den TDA-Gemischen fremden Hilfsstoffen mit einem Siedepunkt von über 290°C das m-TDA von dem TDA-Rückstand abgetrennt werden kann, wobei ein Rückstands/Hilfsstoff-Gemisch mit einem m-TDA-Anteil von 1 - 5 Gew.-% erhalten wird.

EP 794 170 B1 nennt das in EP 659 173 B1 offenbarte Verfahren nachteilig, da das Verhältnis von Rückstand zu Hilfsstoffen bestenfalls nur 1 : 2 beträgt und neben den Kosten für die Hilfsstoffe und deren Entsorgung auch der große Energieaufwand zur Erreichung der geforderten Sumpftemperatur von 290°C, die zur vollständigen Abtrennung des m-TDA erforderlich ist, berücksichtigt werden muss. EP 794 170 B1 offenbart an Stelle eines den TDA-Gemischen fremden Hilfsstoffes vielmehr o-TDA als Schleppmittel einzusetzen. Dadurch kann ein rückstandshaltiger Strom bestehend aus 50 Gew.-% Rückstand, 40 Gew.-% o-TDA und 10 Gew.-% m-TDA-Isomere erhalten werden, der zur thermischen Verwertung gegeben wird. Der im rückstandshaltigen Strom verbleibende m-TDA-Gehalt von 10 Gew.-% stellt einen signifikanten wirtschaftlichen Verlust dar.

Die Herstellung eines im technischen Sinne reinen m-TDAs durch Rückstandsabtrennung aus m-TDA unter Einsatz von o-TDA als Schleppmittel ist ebenfalls Gegenstand von WO 2002/048075 A1. Bei dem in WO 2002/048075 A1 offenbarten Verfahren wird das m-TDA in dem rückstandshaltigen Strom unter Einsatz einer Strippkolonne gegen o-TDA ausgetauscht und nicht durch einfaches Mischen und Destillieren wie in EP 794 170 B1. Dadurch kann der m-TDA-Gehalt im rückstandshaltigen Strom auf unter 0,9 Gew.-%, teilweise auf Werte unterhalb der Nachweisgrenze abgesenkt werden.

Im Gegenzug wird ein m-TDA erhalten, das im Wesentlichen aus den 2,4- und 2,6-TDA-Isomeren besteht und nur noch ca. 0,1 Gew.% (1000 ppm) o-TDA und nur ca. 0,1 Gew.-% TDA-Rückstand enthält. Der Nachteil dieses Verfahrens ist der erforderliche Mehraufwand an Apparaten und Energie.

EP 1 746 083 A1 löst die Erfordernis von einem Mehraufwand an Apparaten dadurch, dass das Roh-TDA in einer Trennwandkolonne destillativ in wenigstens vier Produktströme P1 - P4 aufgetrennt wird, wobei der Produktstrom P1 ein Leichtsieder enthaltender Strom ist, und der Produktstrom P2 ein o-TDA enthaltender Strom ist, und der Produktstrom P3 ein m-TDA enthaltender Strom ist, und der Produktstrom P4 ein Hochsieder und m-TDA enthaltender Produktstrom ist. Durch dieses Verfahren kann beispielsweise ein Produktstrom P3 erhalten werden, der neben m-TDA noch 0,59 Gew.-% p-TDA und nur noch 0,1 Gew.% o-TDA enthält und im technischen Sinne rückstandsfrei ist. Aus dem Produktstrom P4 der Trennwandkolonne kann in einem zusätzlichen Apparat m-TDA abgetrennt werden. Dies kann durch destillative Methoden im Sinne des oben genannten Stands der Technik erfolgen oder beispielsweise durch den Einsatz eines Knetertrockners, der unter Hitze im Vakuum betrieben wird.

Gemäß dem Stand der Technik sind mithin Verfahren bekannt, ein Roh-TDA, wie es großtechnisch aus der Hydrierung von DNT erhalten wird, in ein im technischen Sinne rückstandsfreies m-TDA zu überführen. Weiterhin empfiehlt der Stand der Technik (EP 659 173 B1), dass nur rückstandsfreies m-TDA in die Reaktion mit gasförmigem Phosgen eingesetzt werden sollte.

Der Fachmann entnimmt somit dem Stand der Technik die allgemeine Lehre, dass er das nach der Rückstandsabtrennung erhaltene technisch reine m-TDA gemäß den Methoden nach dem Stand der Technik verdampfen und somit vollständig der Gasphasenphosgenierung zuführen kann.

Überraschend wurde jedoch gefunden, dass sich diese aus dem Stand der Technik ergebende und hinsichtlich der Stoffstromführung äußerst vorteilhafte Verfahrensweise nicht bewährt hat, sondern sich im Gegenteil für die wirtschaftliche Durchführung der Gasphasenphosgenierung von m-TDA als nicht anwendbar erweist.

Die vollständige Überführung eines technisch reinen m-TDA in die Gasphase zum Zwecke der Phosgenierung in der Gasphase führt überraschenderweise nicht zu der für die Gasphasenphosgenierung erforderlichen Reinheit des dampfförmigen TDAs, sondern zu einer starken Bildung von Ammoniak. Diese Ammoniakfreisetzung während der vollständigen Verdampfung von m-TDA führt sowohl in der nachfolgenden Phosgenierungsreaktion als auch in den nachgeschalteten Rohrleitungen und Apparaten zu Ablagerungen von Ammoniumchlorid. Diese Anlagen müssen dann relativ häufig gereinigt werden, wobei entsprechende Produktionsverluste entstehen. Des weiteren wurde gefunden, das ein gemäß dem Stand der Technik von seinem Rückstand befreites m-TDA, das im technischen Sinne als frei von TDA-Rückstand bezeichnet werden kann, nie vollständig rückstandsfrei ist, da sich gewisse Rückstandsmengen bei thermischer Belastung erneut bilden. Speziell bei der nach dem Stand der Technik empfohlenen vollständigen Überführung des m-TDAs in die Gasphase führt dieser Rückstand zu Ablagerungen in den Verdampfersystemen und damit zu einer begrenzten Standzeit der Verdampfersysteme bzw. der Anlagen zur Phosgenierung von m-TDA in der Gasphase.

Aufgabe der vorliegenden Erfindung war es daher, ein einfaches Verfahren zur Herstellung von m-TDI durch Phosgenierung von m-TDA in der Gasphase mit einer m-TDA-Verdampfung bereitzustellen, die sich durch eine geringe Ammoniakfreisetzung bei gleichzeitig hoher Standzeit der Verdampfungsapparatur auszeichnet und damit ein verringertes Auftreten von störenden Feststoffen und den damit verbundenen Anbackungen, Verstopfungen und Stillstandszeiten gewährleistet und sich somit durch eine wesentlich erhöhte Betriebstundenzahl auszeichnet.

Die dieser Erfindung zugrunde liegende Aufgabe konnte dadurch gelöst werden, dass zum einen das zum Zwecke der Phosgenierung in der Gasphase zu verdampfende m-TDA einen möglichst geringen Gehalt an Ammoniakfreisetzung begünstigenden Komponenten enthalten darf. Selbstverständlich sollte das zum Zwecke der Phosgenierung in der Gasphase zu verdampfende m-TDA auch möglichst wenig physikalisch gelösten Ammoniak enthalten.

Art und Gehalt der im zu verdampfenden m-TDA enthaltenen Verunreinigungen beeinflussen wesentlich den Grad der Ammoniakfreisetzung während der Verdampfung. Verdampft das m-TDA, so steigt die Konzentration der Verunreinigungen an und das Maß der Ammoniakfreisetzung vergrößert sich noch. Unerwünschte Isomere des m-TDA sind in diesem Sinne keine Verunreinigungen. Verunreinigungen, die eine verstärkte Freisetzung von Ammoniak begünstigen, sind TDA-Rückstand, cycloaliphatische Amine und Schwermetalle. Schwermetalle, insbesondere Nickel, tragen im besonderen Maße zu einer starken Ammoniakfreisetzung bei (Beispiel 1) und reichern sich bei der Verdampfung von m-TDA im Sumpf des Verdampfers an.

Zum anderen wurde überraschend gefunden, dass die Abtrennung der die Ammoniakfreisetzung begünstigenden Verunreinigungen aus dem m-TDA alleine keine ausreichende Maßnahme zur Erfüllung der dieser Erfindung zugrunde liegenden Aufgabe ist. Denn aus technisch reinem m-TDA können sich bei einer entsprechenden thermischen Belastung des technisch reinen m-TDAs Ammoniakmengen bilden, die immer noch ausreichen, um signifikante Ablagerungen im Verfahren der Gasphasenphosgenierung zu verursachen. Diese Beobachtung erklärt sich dadurch, dass sich gewisse Rückstandsmengen bei thermischer Belastung erneut bilden (Beispiel 2).

Die Reduktion der Ammoniakfreisetzung auf ein technisch vorteilhaftes Maß im Hinblick auf eine wesentliche Erhöhung der Betriebsstundenzahl der Gasphasenphosgenierung von m-TDA unter gleichzeitig wesentlich verbesserter Standzeit der eingesetzten Ver-

dampfungssysteme konnte überraschenderweise durch die Kombination dreier Maßnahmen erreicht werden, deren Kombination sich nicht dem Stand der Technik entnehmen lässt. So ist als erste Maßnahme ein m-TDA in die Verdampfung einzusetzen, das sich über den Stand der Technik hinausgehend dadurch auszeichnet, dass es möglichst kleine Gehalte an Ammoniak, TDA-Rückstand, cycloaliphatischen Aminen sowie Schwermetallen enthält. Diese Maßnahme ist mit einer zweiten und dritten Maßnahme zu kombinieren, die die Bildung von Verunreinigungen und Ammoniak aus dem technisch reinen m-TDA während der Erhitzung zum Zwecke der Verdampfung minimieren. Die zweite Maßnahme besteht darin, die Verdampfung des m-TDA so durchzuführen, dass das Verhältnis zwischen der im Verdampfer vorhandenen Flüssigkeitsmenge **V** [kg] und dem den Verdampfer verlassenden Gasstrom $\dot{M}$ [kg/h] einen Wert von kleiner 2 h einnimmt.

$$\frac{V}{\dot{M}} \ < \ 2 \, h$$

mit

**V** = Flüssigkeitsmenge im m-TDA-Verdampfer [Einheit: Masse]; die Flüssigkeitsmenge **V** beinhaltet dabei die im Verdampfer vorhandene Flüssigkeitsmenge in [kg], die beispielsweise im Falle von Umpump- oder Kreislaufverdampfern auch die Flüssigkeitsmenge im Umpump bzw. im Kreislauf beinhaltet; und

**M** = der den Verdampfer verlassenden Gasstrom **M** [Einheit: Masse/Zeit].

Zusätzlich wird in einer dritten Maßnahme das dem Verdampfer zugeführte m-TDA nicht vollständig verdampft, sondern immer ein Anteil von mindestens 0,1 Gew.-% aus dem Verdampfer ausgeschleust und dieser verbleibende Anteil nicht auf den Reaktor zur Umsetzung des m-TDA mit Phosgen gegeben.

Gegenstand der Erfindung ist daher ein Verfahren zur Herstellung von meta-Toluylendiisocyanat durch Umsetzung von meta-Toluylendiamin mit Phosgen in der Gasphase, bei dem das meta-Toluylendiamin und das Phosgen getrennt voneinander gasförmig einem Reaktor zugeführt werden, wobei das gasförmige meta-Toluylendiamin durch Verdampfung in mindestens einem Verdampfer erzeugt wird, dadurch gekennzeichnet, dass

a) das dem Verdampfer zugeführte flüssige meta-Toluylendiamin weniger als 0,5 Gew.-%, bevorzugt zwischen 0,01 Gew.-% und 0,1 Gew.-%, bezogen auf das Gewicht an meta-Toluylendiamin, an Toluylendiamin-Rückstand, und in der Summe weniger als 0,2 Gew.-%, bezogen auf das Gewicht an meta-Toluylendiamin, an Ammoniak und cycloaliphatischen Aminen, enthält, und

b) das dem Verdampfer zugeführte flüssige meta-Toluylendiamin weniger als 20 ppm, bevorzugt zwischen 0,05 ppm und 10 ppm, bezogen auf das Gewicht an meta-Toluylendiamin, an Schwermetallen enthält, und

c) das Verhältnis zwischen der im Verdampfer vorhandenen Flüssigkeitsmenge **V** [kg] und dem den Verdampfer verlassenden Gasstrom **M** [kg/h] einen Wert von kleiner 2 h einnimmt, und

d) das dem Verdampfer zugeführte meta-Toluylendiamin teilweise verdampft wird, wobei ein Anteil des meta-Toluylendiamin von mindestens 0,1 Gew.-%, bezogen auf das Gewicht an meta-Toluylendiamin, nicht verdampft wird, und

e) der unverdampfte Anteil des meta-Toluylendiamin dem Reaktor nicht zugeführt wird.

Besonders bevorzugt betrifft die Erfindung ein Verfahren, bei dem das dem Verdampfer zugeführte meta-Toluylendiamin

a1) weniger als 50 ppm, bevorzugt zwischen 0,1 und 20 ppm, bezogen auf das Gewicht an meta-Toluylendiamin, an Ammoniak und

a2) weniger als 0,1 Gew.%, bevorzugt zwischen 0,01 Gew.-% und 0,05 Gew.-%, bezogen auf das Gewicht an meta-Toluylendiamin, an cycloaliphatischen Aminen enthält.

Angaben in ppm beziehen sich dabei auf das Gewicht, d.h. 1000 ppm entsprechen 0,1 Gew.%. Die angegebenen Grenzwerte für den Gehalt an Schwermetallen, Ammoniak, cycloaliphatischen Aminen und TDA-Rückstand in dem m-TDA, das dem Verdampfer zugeführt wird, gelten zum Zeitpunkt des Eintritts des m-TDA in den Verdampfer. In der Praxis sollten etwaige genommene Proben möglichst schnell, d.h. möglichst innerhalb von 60 min, zur Analyse präpariert werden.

In einer bevorzugten Ausführung werden die in das erfindungsgemäße Verfahren eingesetzten Toluylendiamine durch katalytische Hydrierung von entsprechenden Dinitrotoluolen erhalten. Die Hydrierung kann unter Mitverwendung von Lösungsmitteln wie Methanol, Ethanol oder Isopropanol aber auch ohne solche Fremdlösungsmittel erfolgen. Die Hydrierung kann mit Hilfe von im Reaktionsgemisch dispergierten Katalysatoren durchgeführt werden, die dann durch Sedimentation oder Filtration abgetrennt und gegebenenfalls in den Prozess zurückgeführt werden. Als Katalysator können dotierte oder undotierte Raney-Nickel-Katalysatoren oder auch Metallkatalysatoren, die beispielsweise Iridium, Kobalt, Kupfer, Palladium oder Platin enthalten, verwendet werden. Bei der technischen Hydrierung von Dinitrotoluol (DNT) zu Toluylendiamin (TDA) wird als Produkt ein TDA-Rohgemisch erhalten, das neben dem Zwangskoppelprodukt Wasser aus mehreren organischen Verbindungen besteht, die sich im Wesentlichen aus 92 bis 96 Gew.-% der m-TDA-Isomeren (2,4- und 2,6-TDA), aus weniger als 1 Gew.-% des p-TDA-Isomeren (2,5-TDA), 3 bis 5 Gew.-% Leichtsieder und 0,2 bis 2 Gew.-% Hochsieder zusammen setzen, wobei sich die Prozentsätze jeweils zu 100 Gew.-% ergänzen. Die Gruppe der Hochsieder enthält auch oligomere, oft farbige Spezies, die durch oxidative Kupplung zweier TDA-Isomeren gebildet und im Allgemeinen als TDA-Rückstand bezeichnet werden. Eine Definition des Begriffs Rückstand im Sinne der Erfindung ist oben gegeben.

Das in der Hydrierung erhaltene TDA-Rohgemisch wird zur weiteren Verwendung gereinigt. Der Reinigungsprozess wird bevorzugt destillativ durchgeführt, wobei das Reaktionswasser sowie Leichtsieder wie Ammoniak, Hexahydro-Toluidin, Hexahydro-Toluylendiamin und gegebenenfalls Lösemittel teilweise oder vollständig entfernt werden. Diese Wasser-, Leichtsieder- und gegebenenfalls auch Lösemittel-Abtrennung kann ein- oder mehrstufig erfolgen. Bevorzugt wird daran anschließend eine destillative Entfernung eines oder mehrerer der o-TDA-Isomere durchgeführt, wobei diese destillative o-TDA-Abtrennung ein- oder mehrstufig erfolgen kann. Bevorzugt erfolgt nach diesen Destillationsschritten eine weitere Aufkonzentrierung des m-TDAs dadurch, dass die Hochsieder aus dem verbliebenen m-TDA abgetrennt werden und so ein m-TDA Produktstrom erhalten wird, der im technischen Sinne rückstandsfrei ist.

In einer bevorzugten Ausführungsform erfolgt nach der oben dargestellten weitgehenden Wasser-, Leichtsieder- und ggf. Lösungsmittelentfernung die Aufbereitung des m-TDAs zu einem im Sinne der vorliegenden Erfindung technisch reinen m-TDA durch die Abtrennung von Leichtsiedern einerseits und Hochsiedern andererseits in einer Trennwandkolonne. Unter einem technisch reinen m-TDA im Sinne der vorliegenden Erfindung wird dabei ein m-TDA verstanden, welches die Anforderungen der Merkmale a) und b) hinsichtlich der Reinheit des erfindungsgemäßen Verfahrens erfüllt. Die Reinigung wird besonders bevorzugt in einer Trennwandkolonne durchgeführt, in der eine Trennwand in Kolonnenlängsrichtung unter Ausbildung eines oberen gemeinsamen Kolonnenbereichs, eines ggf. vorhandenen unteren gemeinsamen Kolonnenbereichs, eines Zulaufteils mit Verstärkungsteil und Abtriebsteil sowie eines Entnahmeteils mit Verstärkungsteil und Abtriebsteil angeordnet ist, welches folgende Schritte umfasst:

A) Zufuhr des Eduktstroms in den Zulaufteil der Trennwandkolonne

B) Abziehen einer Leichtsiederfraktion über den Kolonnenkopf

C) Abziehen von TDA über einen Seitenabzug im Entnahmeteil der Trennwandkolonne

D) Abziehen einer Hochsiederfraktion über den Kolonnensumpf

Der Zulauf der Trennwandkolonne enthält im wesentlichen (d.h. bevorzugt zu mindestens 75 Gew.-%, besonders bevorzugt zu mindestens 87 Gew.-%, ganz besonders bevorzugt zu mindestens 93 Gew.-%) m-TDA und enthält bevorzugt zusätzlich weniger als 10 Gew% o-TDA, weniger als 5 Gew.-% Hochsieder, weniger als 5 Gew.-% Leichtsieder sowie weniger als 5 Gew.-% Wasser. Die Angaben in Gew.-% beziehen sich dabei jeweils auf das Gewicht des Zulaufs zur Trennwandkolonne. Bevorzugt wird die Trennung bei einem absoluten Kopfdruck von zwischen 50 und 2000 mbar, besonders bevorzugt von zwischen 60 und 500 mbar und ganz besonders bevorzugt von zwischen 70 und 200 mbar durchgeführt. Die Kolonne hat im Abtriebsteil des Entnahmeteils bevorzugt mindestens 5 theoretische Trennstufen. Für die Wirksamkeit des Abtriebsteils des Entnahmeteils muss ein Teil der aus dem Verstärkerteil ablaufenden Flüssigkeit auf den Abtriebsteil gegeben werden. Die Menge wird man dabei so wählen, dass sich ein Gehalt an Hochsiedern im aus dem Entnahmeteil abgezogenen m-TDA von weniger als 0,1 Gew.-%, bezogen auf das Gewicht des abgezogenen m-TDA, einstellt.

Dabei können als Trennhilfe alle dem Fachmann geläufigen Einbauten wie Sieb-, Glocken- oder Ventilböden oder geordnete oder ungeordnete Packungen eingesetzt werden. Dabei sollte der Druckverlust durch die Trennhilfe klein gehalten werden; bevorzugt beträgt der Druckverlust weniger als 150 mbar und besonders bevorzugt weniger als 100 mbar. Füllkörperschüttungen und geordnete Packungen weisen bevorzugt eine spezifische Oberfläche von 100 bis 500 und besonders bevorzugt 200 bis 350 $m^2/m^3$ auf. Die Sumpftemperatur wird durch die Gehalte an Hochsiedern und den Druckverlust in der Kolonne bestimmt; bevorzugt wird man die Betriebsbedingungen der Kolonne so wählen, dass sich Sumpftemperaturen von weniger als 260°C und besonders bevorzugt von weniger als 240°C ergeben.

Eine weitere bevorzugte Ausführungsform zur Herstellung eines im Sinne der vorliegenden Erfindung technisch reinen m-TDAs beinhaltet die Reinigung des m-TDAs in einer Anordnung aus Kolonnen, bei der eine gemeinsame Kopfkolonne mit einer Zulaufkolonne, einer Entnahmekolonne und ggf. einer gemeinsamen Sumpfkolonne verbunden sind. Dabei werden die Dämpfe aus Zulauf- und Entnahmekolonne in die Kopfkolonne geleitet, während der Flüssigkeitsablauf aus der Kopfkolonne auf Zulauf- und Entnahmekolonne verteilt wird. Die Dämpfe der ggf. vorhandenen Sumpfkolonne werden in Zulauf- und Entnahmekolonne geleitet, während die Flüssigkeitsabläufe aus Zulauf- und Entnahmekolonne auf die ggf. vorhandene Sumpfkolonne geleitet werden.

Eine weitere bevorzugte Ausführungsform zur Herstellung eines im Sinne der vorliegenden Erfindung technisch reinen m-TDAs beinhaltet die Reinigung des m-TDAs in einer Anordnung aus Kolonnen, in der zunächst das Roh-TDA von Leichtsiedern und o-TDA befreit und das so erhaltene Sumpfprodukt in einer weiteren Kolonne von Hochsiedern befreit wird. Die Kolonne hat im Verstärkerteil bevorzugt mindestens 5 theoretische Trennstufen. Der Rücklauf auf die Kolonne ist dabei so zu wählen, dass sich im Kopfprodukt ein Gehalt von Hochsiedern von weniger als 0,1 Gew.%, bezogen auf das Gewicht des Kopfprodukts, einstellt. Auch bei Wahl von schonenden Verdampfungsbedingungen kann es zur Bildung von Rückstand im Verdampfer oder in der Kolonne kommen, wodurch Ammoniak freigesetzt wird. Da bei dieser Ausführungsform das m-TDA als Kopfprodukt erhalten wird, sind die Bedingungen unter denen die Kondensation erfolgt, so zu wählen, dass ein Produkt mit bevorzugt weniger als 50 ppm gelöstem Ammoniak erhalten wird. Dies wird durch eine ausreichend hohe Kondensationstemperatur, bei der nur wenig Ammoniak im Kondensationsprodukt gelöst wird, ermöglicht. Alternativ oder zusätzlich kann beispielsweise auch ein Inertgas in diesen Destillationsschritt gegeben werden, das als Schleppmittel für Ammoniak wirkt und einen Abtransport des Ammoniak über den Entgasungsweg der Kolonne bewirkt.

Die skizzierten Reinigungen des TDA-Rohgemisches sind so durchzuführen, dass das erhaltene und im technischen Sinne reine m-TDA in der Summe weniger als 0,5 Gew.-% TDA-Rückstand, bevorzugt weniger als 0,1 Gew.-% TDA-Rückstand und in der Summe weniger als 0,2 Gew.-% Verunreinigungen, bevorzugt weniger als 0,1 Gew.-% cycloaliphatische Amine, weniger als 50 ppm Ammoniak und weniger als 10 ppm Schwermetalle, jeweils bezogen auf das Gewicht an m-TDA, enthält. Bei der Durchführung des erfindungsgemäßen Verfahrens ist ebenfalls die Lagerung des m-TDAs zu berücksichtigen. Bei der Lagerung des im technischen Sinne reinen m-TDAs oder durch Kontakt mit Luft kann sich TDA-Rückstand bilden. Somit enthält das im technischen Sinne reine m-TDA üblicherweise geringe Mengen an TDA-Rückstand, d.h. der Gehalt an TDA-Rückstand ist üblicherweise größer als 0 Gew.-% und bevorzugt kleiner als 0,1 Gew.-%.

Zum Zwecke der Gasphasenphosgenierung wird das durch eine der vorgenannten bevorzugten Ausführungsformen erhaltene meta-Toluylendiamin in mindestens einem Verdampfer verdampft sowie auf Temperaturen von 200°C bis 600°C, bevorzugt 200°C bis 500°C, besonders bevorzugt 250°C bis 450°C erhitzt und gegebenenfalls verdünnt mit einem Inertgas wie $N_2$, He, Ar oder mit den Dämpfen eines inerten Lösungsmittels, z.B. aromatischen Kohlenwasserstoffen ggf. mit Halogensubstitution, wie z.B. Chlorbenzol oder ortho-Dichlorbenzol, dem Reaktionsraum zugeführt.

Bei dem erfindungsgemäßen Verfahren wird der dem Verdampfer zugeführte Strom, der durch eine der vorgenannten bevorzugten Ausführungsformen erhaltene wurde, teilweise, d.h. bevorzugt zu mindestens 70 Gew.-%, besonders bevorzugt zu mindestens 90 Gew.-%, ganz besonders bevorzugt zu mindestens 95 Gew.-%, bezogen auf das Gewicht des m-TDA, verdampft und zu mindestens 0,1 Gew.-%, bevorzugt zu mindestens 0,5 Gew.-%, besonders bevorzugt zu mindestens 1,0 Gew.-%, bezogen auf das Gewicht des m-TDA, nicht verdampft. In diesem unverdampften Teilstrom reichern sich die mit dem m-TDA eingetragenen Hochsieder sowie die durch die Erhitzung des m-TDA gebildeten Verunreinigungen, die die Ammoniakfreisetzung begünstigen, an. Die nicht verdampfte Teilmenge wird aus dem Verdampfer kontinuierlich oder chargenweise ausgeschleust und nicht dem Gasphasenreaktor zugeführt. Bevorzugt wird der unverdampfte Teilstrom teilweise oder vollständig wieder der oben beschriebenen destillativen Aufarbeitung des Roh-TDA zugeführt. In einer weiteren ebenfalls bevorzugten Ausführungsform kann der nicht verdampfte Teilstrom kontinuierlich oder chargenweise aus dem Verdampfer ausgetragen und entsorgt, bevorzugt thermisch entsorgt werden. In einer weiteren, ebenfalls bevorzugten Ausführungsform kann der nicht verdampfte Teilstrom kontinuierlich oder chargenweise aus dem Verdampfer ausgetragen und teilweise, bevorzugt zu 10 bis 90%, zusammen mit dem technisch reinen m-TDA wieder direkt in den Verdampfer eingespeist werden.

Als Verdampfer für die Aminverdampfung können grundsätzlich beliebige geeignete Verdampfer eingesetzt werden. Bevorzugt können Rohrbündelwärmetauscher, Plattenwärmetauscher oder Fallfilmverdampfer, gegebenenfalls mit Umpumpkreislauf, eingesetzt werden. Auch können Mikrowärmetauscher oder Mikroverdampfer wie in WO 2005/016512 A oder in DE 10 2005 036870 A1 beschrieben eingesetzt werden, sofern diese im Sinne der vorliegenden Erfindung nur zur Teilverdampfung des dem Verdampfer zugeführten m-TDA-Stroms eingesetzt werden.

In einer bevorzugten Ausführungsform erfolgt die Verdampfung des meta-Toluylendiamin in einem Fallfilmverdampfer mit Umpump. Das im Fallfilmverdampfer bei Temperaturen oberhalb seines Siedepunktes verdampfte m-TDA wird dem Reaktor zum Zwecke der Gasphasenphosgenierung zugeführt, während der nicht verdampfte Anteil des Verdampferzulaufstroms dem Reaktor nicht zugeführt wird. Der nicht verdampfte Anteil des Verdampferzulaufstroms wird von dem verdampften m-TDA abgeschieden, wobei die Abscheidung ein- oder mehrstufig und durch einfache Kondensation, durch Variation der Strömungsverhältnisse oder gegebenenfalls unter Einsatz von Tröpfchenabscheidern erfolgen kann. Der nicht verdampfte Anteil des Verdampferzulaufstroms wird in der Pumpvorlage des Fallfilmverdampfers gesammelt und kann von dort teilweise zurück auf den Fallfilmverdampfer geführt und / oder ganz oder teilweise zurück in die TDA-Destillationssequenz gegeben werden und / oder ganz oder teilweise ausgeschleust und entsorgt werden. Sofern der nicht verdampfte Anteil teilweise zurück auf den Fallfilmverdampfer geführt wird und / oder ganz oder teilweise zurück in die TDA Destillationssequenz gegeben wird, wird dieses bevorzugt kontinuierlich durchgeführt. Sofern der nicht verdampfte Anteil teilweise zurück auf den Fallfilmverdampfer geführt wird und / oder ganz oder teilweise ausgeschleust und entsorgt wird, kann dieses kontinuierlich oder diskontinuierlich, bevorzugt diskontinuierlich durchgeführt werden. Eine Verringerung der Flüssigkeitsmenge **V** im m-TDA-Verdampfer wirkt sich vorteilhaft auf die Unterdrückung der Bildung von Ammoniak und anderen Verunreinigungen aus. Im Falle des Einsatzes des hier beschriebenen Fallfilmverdampfers mit Umpumpkreislauf und Pumpenvorlage umfasst die Flüssigkeitsmenge **V** die Flüssigkeitsmenge im Fallfilmverdampfer, im Umpumpkreislauf und in der Pumpenvorlage. In allen Ausführungsformen wird das Verhältnis zwischen der im Sumpf des Verdampfers vorgehaltenen Flüssigkeitsmenge **V** entsprechend oben gegebener Definition und dem im Verdampfer in die Gasphase überführten Strom **Ṁ** so eingestellt, dass es einen Wert von kleiner 2 h einnimmt. Dies gewährleistet, dass der dem Verdampfer zugeführte Strom an im Sinne dieser Erfindung technisch reinem meta-Toluylendiamin innerhalb des Verdampfers hinreichend wenig Ammoniak freisetzt.

Weiterhin bevorzugt erfolgt die Verdampfung und Überhitzung des m-TDA mehrstufig, um unverdampfte Tröpfchen im dampfförmigen TDA-Strom zu vermeiden. Insbesondere bevorzugt sind mehrstufige Verdampfungs- und Überhitzungsschritte, in denen zwischen den Verdampfungs- und Überhitzungssystemen Tröpfchenabscheider eingebaut sind und / oder die Verdampfungsapparaturen auch die Funktion eines Tröpfchenabscheiders besitzen. Geeignete Tröpfchenabscheider sind z.B. beschrieben in "Droplet Separation", A. Bürkholz, VCH Verlagsgesellschaft, Weinheim - New York - Basel - Cambridge, 1989. Besonders bevorzugt sind Tröpfchenabscheider die einen geringen Druckverlust verursachen. Ganz besonders bevorzugt wird das verdampfte Amin über zumindest einen Nacherhitzer auf die gewünschte Einsatztemperatur gebracht, der auch als Tröpfchenabscheider fungiert. Insbesondere bevorzugt hat dieser Nacherhitzer einen Flüssigkeitsablauf um die stetige Entleerung des Abscheiders zu gewährleisten.

Nach Verlassen des in Strömungsrichtung letzten Überhitzers wird das auf seine Solltemperatur vorgeheizte dampfförmige Amin mit einer mittleren Verweilzeit von bevorzugt 0,01 bis 60 s, ganz besonders bevorzugt von 0,01 bis 30 s, insbesondere bevorzugt 0,01 bis 15 s, dem Reaktor bzw. dessen Mischeinrichtung zur Umsetzung zugeführt, dabei wird über technische Maßnahmen, z.B. eine ausreichende Isolierung zur Vermeidung von Abstrahlungsverlusten, der Gefahr einer erneuten Tröpfchenbildung begegnet. Durch die Erzeugung eines im Wesentlichen tröpfchenfreien dampfförmigen Ausgangsaminstromes vor Eintritt in den Reaktor wird die Reaktorlaufzeit deutlich erhöht. Unter einem im Wesentlichen tröpfchenfreien dampfförmigen Ausgangsaminstrom wird verstanden, dass die dampfförmigen Amine im wesentlichen keine Tröpfchen an unverdampften Aminen enthalten, das heißt, dass maximal 0,5 Gew.-% des Amins, besonders bevorzugt maximal 0,05 Gew.-% des Amins, bezogen auf das Gesamtgewicht an Amin, in der Form von unverdampften Tröpfchen vorliegt und der restliche Teil des Amins dampfförmig vorliegt. Ganz besonders bevorzugt enthalten die dampfförmigen Amine keine Tröpfchen an unverdampften Aminen. Die ggf. vorliegenden Tröpfchen an unverdampften Amin im ansonsten dampfförmigen Aminstrom sind im Sinne der vorliegenden Erfindung nicht dem unverdampften Anteil des Amins gemäß Merkmal d) des erfindungsgemäßen Verfahrens zuzurechnen. Unabhängig von ggf. mitgerissenen Amintröpfchen im dampfförmigen Aminstrom wird in dem erfindungsgemäßen Verfahren ein Anteil von mindestens 0,1 Gew.-% an meta-Toluylendiamin, bezogen auf das Gewicht an meta-Toluylendiamin, nicht verdampft und gemäß Merkmal e) des erfindungsgemäßen Verfahrens dem Reaktor nicht zugeführt.

Die abgeschiedenen Flüssigkeitsmengen werden gegebenenfalls mit dem nicht verdampften TDA aus dem Verdampfer zusammengeführt.

Die Verdampfer und / oder Überhitzer sowie die Rohrleitungen zur Erzeugung des dampfförmigen m-TDA-Stroms zum Gasphasenreaktor können aus einem beliebigen metallischen Werkstoff gefertigt sein, z.B. Stahl, Edelstahl, Titan, Hastelloy, Inconel oder anderen metallischen Legierungen. Bevorzugt werden metallische Werkstoffe mit einem geringen Nickel-Anteil verwendet.

Der Teilstrom des verdampften m-TDA wird, gegebenenfalls verdünnt mit einem Inertgas oder mit den Dämpfen eines inerten Lösungsmittels, auf Temperaturen von 200°C bis 600°C erhitzt und wird mit einem Phosgenstrom, der, gegebenenfalls verdünnt mit einem Inertgas oder mit den Dämpfen eines inerten Lösungsmittels, getrennt von dem Aminstrom ebenfalls auf Temperaturen von 200°C bis 600°C erhitzt wurde, kontinuierlich in einem Rohrreaktor mit gleichbleibendem oder sich veränderndem Querschnitt unter Aufrechterhaltung einer turbulenten Strömung innerhalb einer mittleren Verweilzeit von 0,5 bis 15 Sekunden zur Reaktion gebracht. Das den Reaktionsraum verlassende Gasgemisch wird durch mindestens ein inertes Lösungsmittel und / oder durch mindestens ein Gemisch aus inertem Lösungsmittel und Diisocyanat geleitet, wobei das Lösungsmittel oder das Gemisch bei einer Temperatur oberhalb der Zersetzungstemperatur des dem Diamin entsprechenden Carbamidsäurechlorids gehalten werden. Das sich dabei in dem inerten Lösungsmittel lösende Diisocyanat wird einer destillativen Aufarbeitung unterzogen.

In einer weiteren bevorzugten Variante der Erfindung wird der nicht verdampfte Teilstrom, der ganz oder teilweise ausgeschleust und entsorgt oder ganz oder teilweise ausgeschleust und in die TDA-Destillationssequenz gegeben wird, nach der Ausschleusung und vor der Entsorgung oder Zuführung in die TDA-Destillationssequenz gekühlt, und zwar auf eine Temperatur unterhalb der Siedetemperatur des TDAs in dem Verdampfer, bevorzugt auf Temperaturen unterhalb von 260°C, insbesondere bevorzugt auf Temperaturen unterhalb von 200°C. Dabei unterschreitet die Temperatur jedoch nicht 100°C, besonders bevorzugt nicht 130°C. Die Abkühlung kann in allen gängigen Wärmetauschern wie z.B. in Rohrbündelwärmetauschern, oder Plattenwärmetauschern erfolgen. Als Kühlmittel kann Wasser, Kondensat, sekundäre Kühlmittelkreisläufe, Luft oder auch andere aufzuheizende Ströme in der Anlage verwendet werden. Insbesondere bevorzugt erfolgt die Kühlung gegen andere aufzuheizende Ströme, besonders bevorzugt gegen TDA.

Eine weitere Ausführungsform zur Herstellung eines m-TDAs, das sich im Sinne der vorliegenden Erfindung durch eine geringe Ammoniakfreisetzung bei der Verdampfung auszeichnet, beinhaltet die thermische Behandlung eines von Leichtsiedern und o-TDA befreiten m-TDAs, das aber noch Hochsieder enthalten kann, bei hohen Temperaturen für eine längere Verweilzeit. Bevorzugt betragen diese Temperaturen 100 bis 350°C, besonders bevorzugt 130 bis 330°C und ganz besonders bevorzugt 200 bis 320°C während die Verweilzeit bei dieser Temperatur länger als 15 Minuten, besonders bevorzugt 20 Minuten bis 10 Tage, ganz besonders bevorzugt 30 Minuten bis 4 Stunden ist. Dies ist insofern vorteilhaft, als dass die zur Bildung von Ammoniak führenden Reaktionen dabei in nennenswertem Umfang ablaufen und der dabei entstehende Ammoniak abgetrennt werden kann. Das nach Abtrennung des Ammoniaks erhaltene m-TDA wird bei der anschließenden Verdampfung eine geringere Neigung zur Abspaltung von Ammoniak haben.

Die Erfindung betrifft somit auch ein Verfahren zur Herstellung von meta-Toluylendiisocyanat durch Umsetzung von meta-Toluylendiamin mit Phosgen in der Gasphase, bei dem das meta-Toluylendiamin und das Phosgen getrennt voneinander gasförmig einem Reaktor zugeführt werden, wobei das gasförmige meta-Toluylendiamin durch Verdampfung in mindestens einem Verdampfer erzeugt wird, dadurch gekennzeichnet, dass das dem Verdampfer zugeführte flüssige meta-Toluylendiamin

a) in der Summe weniger als 0,2 Gew.-%, bezogen auf das Gewicht an meta-Toluylendiamin, an Ammoniak und cycloaliphatischen Aminen enthält, und

b) weniger als 20 ppm Schwermetalle enthält, und

c) das dem Verdampfer zugeführte meta-Toluylendiamin zuvor bei mindestens 100°C für mindestens 15 Minuten thermisch behandelt wird und der dabei entstandene Ammoniak abgetrennt wird.

Diese Ausführungsform des erfindungsgemäßen Verfahrens hat mit der oben beschriebenen Variante gemein, dass das dem Verdampfer zugeführte meta-Toluylendiamin die spezifizierte Reinheit bzgl. Ammoniak, cycloaliphatischen Aminen und Schwermetallen aufweist. Die Reinheit des m-TDA allein ist jedoch nicht ausreichend und muss daher mit mindestens einer weiteren Maßnahme kombiniert werden. In der hier dargestellten Variante ist es die Temperung des meta-Toluylendiamin vor der Verdampfung. Während der Temperung kann ein Rückstandsgehalt von mehr als 0,5 Gew.-% toleriert werden. Nach dieser Temperung ist eine exakte Einstellung des Verhältnisses zwischen der im Verdampfer vorhandenen Flüssigkeitmenge $V$ [kg] und dem den Verdampfer verlassenden Gasstrom $M$ [kg/h] auf einen Wert von kleiner 2 h nicht mehr zwingend erforderlich.

**Beispiele**

Die Zusammensetzung von TDA-Isomerengemischen und ihr Gehalt an **cycloaliphatischen Aminen** werden üblicherweise mit gaschromatographischen Methoden ermittelt, die dem Fachmann experimentell leicht zugänglich sind. Beispielsweise ist die Methode von Willeboordse *et al.* dazu geeignet (Willeboordse, F.; Quick, Q.; Bishop, E.T. "Direct gas chromatographic analysis of isomeric diaminotoluenes" Analytical Chemistry 1968, 40 (10), 1455-1458).

Der Gehalt an **TDA-Rückstand** wird üblicherweise durch Rückstandsdestillation ermittelt, wobei der Massenanteil des TDA-Rückstandes in einer Probe durch Wägung ermittelt wird, bevor und nachdem TDA-Isomere, cycloaliphatische Amine und gegebenenfalls weitere Leichtsieder abdestilliert wurden.

Der Gehalt an **Schwermetallen** kann durch dem Fachmann bekannte Methoden mittels Atomadsorptionsspektroskopie ermittelt werden.

Der Gehalt an physikalisch gelöstem Ammoniak wird aus einer flüssigen m-TDA-Probe bestimmt, wie sie aus dem Zulaufstrom des Verdampfers bzw. durch vollständige Kondensation eines Teilstroms des den Verdampfer verlassenden Gasstroms erhalten werden kann. Die Probe wird in einem Edelstahl-Autoklaven bei 140°C für 1 h mit einem Stickstoffstrom ausgeblasen und so der **physikalisch gelöste Ammoniak** aus dem m-TDA ausgetragen. Der Stickstoffstrom wird außerhalb des Autoklaven von mitgerissenem TDA befreit und der Ammoniak in einer Gaswaschflasche in einem Überschuss verdünnter Schwefelsäure neutralisiert. Durch Rücktitration des Schwefelsäuregehaltes mit 0,1 N Natronlauge wurde der Verbrauch an Schwefelsäure und damit die Menge an physikalisch gelöstem Ammoniak bestimmt.

**Beispiel 1** (nicht erfindungsgemäß)

Verwendung von m-TDA, das mit Nickelsalz aufgestockt wurde

Um den Einfluss von Schwermetallionen auf die Ammoniakfreisetzung zu zeigen wurde in Beispiel 1 603 g eines m-TDA eingesetzt, wie es durch ein TDA-Verfahren ohne Rückstandsabtrennung bereitgestellt wird und das zusätzlich mit 0,12 g Nickel in Form eines Nickel(II)-Salzes versetzt wurde, so dass sich eine Nickelionenkonzentration von 200 ppm ergab. Dieses m-TDA zeigte einen Rückstandsgehalt von 1,13 Gew.-% während der Gehalt an cycloaliphatischen Aminen 0,12 Gew.% und der Gehalt an physikalisch gelöstem Ammoniak 25 ppm betrug. Dieses mit Nickelionen versetzte m-TDA wurde für 5 h einer Temperatur von 320°C ausgesetzt und zeigte in diesem Zeitraum eine äußerst starke Ammoniakfreisetzung von 8155 mg/kg m-TDA.

**Beispiel 2** (nicht erfindungsgemäß)

Rückstandsbildung während der Temperung von im technischen Sinne reinem m-TDA.
In Beispiel 2 wurden 599 g m-TDA eingesetzt, das zuvor destilliert und so von Schwermetallen, TDA-Rückstand und cycloaliphatischen Aminen befreit wurde. Die Probe war im technischen Sinne frei von Schwermetallen (< 10 ppm), rückstandsfrei (< 0,05 Gew.-% TDA-Rückstand) und der Gehalt an cycloaliphatischen Aminen konnte auf 0,02 Gew.-% reduziert werden. Der Gehalt an physikalisch gelöstem Ammoniak betrug 31 ppm. Diese Probe wurde für 5 h einer Temperatur von 320°C ausgesetzt und zeigte in diesem Zeitraum eine Ammoniakfreisetzung, die über die Freisetzung des physikalisch gelösten Ammoniaks hinausging, von 89 mg/kg TDA. Nach der Temperung wurde der Rückstandsgehalt erneut bestimmt und ein Anstieg auf 1,01 Gew.-% ermittelt.

**Beispiel 3** (nicht erfingdungsgemäß

Ein Strom bestehend aus 3140 kg/h eines Gemisches enthaltend 1,2 Gew.-% TDA-Rückstand, 50 ppm $NH_3$, 500 ppm cycloaliphatische Amine und 3 ppm Schwermetalle, der Rest im wesentlichen m-TDA, wird einem Verdampfer zugeführt, in dem 2400 kg/h des Gemisches verdampft und einer Gasphasenphosgenierung zugeführt wird. 739 kg/h, also 23,5 Gew.-% des Einlaufstroms werden ausgeschleust und einer anderen Verwendung zugeführt. Der Verdampfer ist als Fallfilmverdampfer mit Pumpvorlage und Umlaufpumpe ausgeführt und wird bei 320°C und 1,2 bar betrieben. Die Pumpvorlage entspricht dem Sumpf des Verdampfers. Das Arbeitsvolumen in der Pumpvorlage beträgt 1,2 m³, was einer im Verdampfer vorgehaltenen Flüssigkeitsmenge von ca. 1020 kg entspricht. Das Verhältnis der im Sumpf des Verdampfers vorgehaltenen Flüssigkeitsmenge zum verdampften Strom beträgt 0,4 h. Der Ammoniakgehalt im Strom zur Gasphasenphosgenierung beträgt ca. 90 ppm, es wird rasche Verstopfung beobachtet.

**Beispiel 4** (nicht erfindungsgemäß)

Ein Strom bestehend aus 2402 kg/h eines im technischen Sinne rückstand- und schwermetallfreien Gemisches enthaltend 1000 ppm cycloaliphatische Amine, 12 ppm Ammoniak, der Rest im wesentlichen m-TDA, wird der Anordnung aus Beispiel 3 zugeführt. Ca. 2400 kg/h werden verdampft, ca. 2 kg/h, also 0,08 Gew.-% des Einlaufstroms werden ausgeschleust und einer anderen Verwendung zugeführt. Das Arbeitsvolumen in der Pumpvorlage beträgt 1,2 m³, was einer im Verdampfer vorgehaltenen Flüssigkeitsmenge von ca. 1020 kg entspricht. Das Verhältnis der im Sumpf des Verdampfers vorgehaltenen Flüssigkeitsmenge zum verdampften Strom beträgt 0,4 h. Der Ammoniakgehalt im Strom zur Gasphasenphosgenierung beträgt ca. 76 ppm, es wird merkliche Verstopfung beobachtet.

**Beispiel 5** (nicht erfindungsgemäß)

Ein Strom bestehend aus 2450 kg/h eines im technischen Sinne rückstand- und schwermetallfreien Gemisches enthaltend 1000 ppm cycloaliphatische Amine, 12 ppm Ammoniak, der Rest im wesentlichen m-TDA, wird der Anordnung aus Beispiel 3 zugeführt. Ca. 2400 kg/h werden verdampft, ca. 50 kg/h, also 2,0 Gew.-% des Einlaufstroms werden ausgeschleust und einer anderen Verwendung zugeführt. Das Arbeitsvolumen in der Pumpvorlage beträgt 6 m³, was einer im Verdampfer vorgehaltenen Flüssigkeitsmenge von ca. 5100 kg entspricht. Das Verhältnis der im Sumpf des Verdampfers vorgehaltenen Flüssigkeitsmenge zum verdampften Strom beträgt 2,1 h. Der Ammoniakgehalt im Strom zur Gasphasenphosgenierung beträgt ca. 137 ppm, es wird rasche Verstopfung beobachtet.

**Beispiel 6** (erfindungsemäß)

Ein Strom bestehend aus 2424 kg/h eines im technischen Sinne schwermetallfreien Gemisches enthaltend 200 ppm cycloaliphatische Amine, 14 ppm Ammoniak und 0,1 Gew.-% Rückstand, der Rest im wesentlichen m-TDA, wird der Anordnung aus Beispiel 3 zugeführt. Ca. 2400 kg/h werden verdampft, ca. 24 kg/h, also 1,0 Gew.-% des Einlaufstroms werden ausgeschleust und einer anderen Verwendung zugeführt. Das Arbeitsvolumen in der Pumpvorlage beträgt 1,2 m³. Das Verhältnis der im Sumpf des Verdampfers vorgehaltenen Flüssigkeitsmenge zum verdampften Strom beträgt 0,4 h. Der Ammoniakgehalt im Strom zur Gasphasenphosgenierung beträgt ca. 30 ppm, es wird eine im Vergleich zu den Beispielen 3 bis 5 niedrigere Verstopfungsneigung und eine erhöhte Betriebsstundenzahl beobachtet.

## Patentansprüche

1. Verfahren zur Herstellung von m-Toluylendüsocyanat durch Umsetzung von meta-Toluylendiamin mit Phosgen in der Gasphase, bei dem das meta-Toluylendiamin und das Phosgen getrennt voneinander gasförmig einem Reaktor zugeführt werden, wobei das gasförmige meta-Toluylendiamin durch Verdampfung in mindestens einem Verdampfer erzeugt wird, **dadurch gekennzeichnet, dass**

   a) das dem Verdampfer zugeführte flüssige meta-Toluylendiamin weniger als 0,5 Gew.-%, bezogen auf das Gewicht an meta-Toluylendiamin, an Toluylendiamin-Rückstand, und in der Summe weniger als 0,2 Gew.-%, bezogen auf das Gewicht an meta-Toluylendiamin, an Ammoniak und cycloaliphatischen Aminen, enthält, und
   b) das dem Verdampfer zugeführte flüssige meta-Toluylendiamin weniger als 20 ppm, bezogen auf das Gewicht an meta-Toluylendiamin, an Schwermetallen enthält, und
   c) das Verhältnis zwischen der im Verdampfer vorhandenen Flüssigkeitsmenge **V** [kg] und dem den Verdampfer verlassenden Gasstrom **M** [kg/h] einen Wert von kleiner 2 h einnimmt, und
   d) das dem Verdampfer zugeführte meta-Toluylendiamin teilweise verdampft wird, wobei ein Anteil des meta-Toluylendiamin von mindestens 0,1 Gew.%, bezogen auf das Gewicht an meta-Toluylendiamin, nicht verdampft wird, und
   e) der unverdampfte Anteil des meta-Toluylendiamin dem Reaktor nicht zugeführt wird.

2. Verfahren nach Anspruch 1, **dadurch gekennzeichnet, dass** das dem zumindest einem Verdampfer zugeführte meta-Toluylendiamin

   a1) weniger als 50 ppm, bezogen auf das Gewicht an meta-Toluylendiamin, an Ammoniak sowie
   a2) weniger als 0,1 Gew.%, bezogen auf das Gewicht an meta-Toluylendiamin, an cycloaliphatischen Aminen enthält.

3. Verfahren nach Anspruch 1, **dadurch gekennzeichnet, dass** in Schritt d) der nicht verdampfte Anteil des meta-Toluylendiamin aus dem Verdampfer durch eine Austrittsöffnung ausgeschleust und anschließend dem Verdampfer durch eine Eintrittsöffnung teilweise wieder zugeführt wird.

4. Verfahren nach Anspruch 1, **dadurch gekennzeichnet, dass** in Schritt d) der nicht verdampfte Anteil des meta-Toluylendiamin aus dem Verdampfer durch eine Austrittsöffnung ausgeschleust und anschließend zumindest teilweise in die Destillation von rohem Toluylendiamin geführt wird, das aus der Hydrierung von Dinitrotoluol erhalten wird.

5. Verfahren zur Herstellung von meta-Toluylendiisocyanat durch Umsetzung von meta-Toluylendiamin mit Phosgen in der Gasphase, bei dem das meta-Toluylendiamin und das Phosgen getrennt voneinander gasförmig einem Reaktor zugeführt werden, wobei das gasförmige meta-Toluylendiamin durch Verdampfung in mindestens einem Verdampfer erzeugt wird, **dadurch gekennzeichnet, dass**

   a) das dem Verdampfer zugeführte flüssige meta-Toluylendiamin in der Summe weniger als 0,2 Gew.-%, bezogen auf das Gewicht an meta-Toluylendiamin, an Ammoniak und cycloaliphatischen Aminen enthält, und
   b) das dem Verdampfer zugeführte flüssige meta-Toluylendiamin weniger als 20 ppm Schwermetalle enthält, und
   c) das dem Verdampfer zugeführte meta-Toluylendiamin zuvor bei mindestens 100°C für mindestens 15 Minuten thermisch behandelt wird und der dabei entstandene Ammoniak abgetrennt wird.

## Claims

1. Process for the preparation of m-toluylene-diisocyanate by reaction of meta-toluylenediamine with phosgene in the gas phase, in which the meta-toluylenediamine and the phosgene are fed separately from one another in gaseous form to a reactor, wherein the gaseous meta-toluylenediamine is generated by vaporization in at least one evaporator, **characterized in that**

   a) the liquid meta-toluylenediamine fed to the evaporator contains less than 0.5 wt.%, based on the weight of meta-toluylenediamine, of toluylenediamine residue, and in total less than 0.2 wt.%, based on the weight of meta-toluylenediamine, of ammonia and cycloaliphatic amines, and
   b) the liquid meta-toluylenediamine fed to the evaporator contains less than 20 ppm, based on the weight of meta-toluylenediamine, of heavy metals, and
   c) the ratio between the amount of liquid V [kg] present in the evaporator and the gas stream M [kg/h] leaving the evaporator assumes a value of less than 2 h, and
   d) the meta-toluylenediamine fed to the evaporator is partly vaporized, a content of the meta-toluylenediamine of at least 0.1 wt.%, based on the weight of meta-toluylenediamine, not being vaporized, and

e) the non-vaporized content of the meta-toluylenediamine is not fed to the reactor.

2. Process according to claim 1, **characterized in that** the meta-toluylenediamine fed to the at least one evaporator contains

a1) less than 50 ppm, based on the weight of meta-toluylenediamine, of ammonia and
a2) less than 0.1 wt.%, based on the weight of meta-toluylenediamine, of cycloaliphatic amines.

3. Process according to claim 1, **characterized in that** in step d) the non-vaporized content of meta-toluylenediamine is sluiced out of the evaporator through an exit opening and is then partly fed to the evaporator again through an intake opening.

4. Process according to claim 1, **characterized in that** in step d) the non-vaporized content of meta-toluylenediamine is sluiced out of the evaporator through an exit opening and is then at least partly fed into the distillation of crude toluylenediamine obtained from the hydrogenation of dinitrotoluene.

5. Process for the preparation of meta-toluylene-diisocyanate by reaction of meta-toluylenediamine with phosgene in the gas phase, in which the meta-toluylenediamine and the phosgene are fed separately from one another in gaseous form to a reactor, wherein the gaseous meta-toluylenediamine is generated by vaporization in at least one evaporator, **characterized in that**

a) the liquid meta-toluylenediamine fed to the evaporator contains in total less than 0.2 wt.%, based on the weight of meta-toluylenediamine, of ammonia and cycloaliphatic amines, and
b) the liquid meta-toluylenediamine fed to the evaporator contains less than 20 ppm of heavy metals, and
c) the meta-toluylenediamine fed to the evaporator is heat treated beforehand at not less than 100°C for at least 15 minutes and the ammonia thereby formed is separated off.

## Revendications

1. Procédé pour la préparation de diisocyanate de m-toluylène par transformation de méta-toluylènediamine avec du phosgène en phase gazeuse, dans lequel la méta-toluylènediamine et le phosgène sont introduits séparément l'un de l'autre sous forme gazeuse dans un réacteur, la méta-toluylènediamine sous forme gazeuse étant générée par évaporation dans au moins un évaporateur, **caractérisé en ce que**

a) la méta-toluylènediamine liquide introduite dans l'évaporateur contient moins de 0,5% en poids, par rapport au poids de méta-toluylènediamine, de résidu de toluylènediamine et, au total, moins de 0,2% en poids, par rapport au poids de méta-toluylènediamine, d'ammoniaque et d'amines cycloaliphatiques et
b) la méta-toluylènediamine liquide introduite dans l'évaporateur contient moins de 20 ppm, par rapport au poids de méta-toluylènediamine, de métaux lourds et
c) le rapport entre la quantité de liquide V [kg] présente dans l'évaporateur et le flux gazeux M [kg/h] quittant l'évaporateur présente une valeur inférieure à 2 h et
d) la méta-toluylènediamine introduite dans l'évaporateur est évaporée au moins partiellement, une partie d'au moins 0,1% en poids de la méta-toluylènediamine, par rapport au poids de la méta-toluylènediamine, n'étant pas évaporée et
e) la partie non évaporée de la méta-toluylènediamine n'étant pas introduite dans le réacteur.

2. Procédé selon la revendication 1, **caractérisé en ce que** la méta-toluylènediamine introduite dans au moins un évaporateur

a1) contient moins de 50 ppm, par rapport au poids de méta-toluylènediamine, d'ammoniaque et
a2) contient moins de 0,1% en poids, par rapport au poids de méta-toluylènediamine, d'amines cycloaliphatiques.

3. Procédé selon la revendication 1, **caractérisé en ce que** la partie de la méta-toluylènediamine non évaporée dans l'étape d) est évacuée de l'évaporateur par une ouverture de sortie et à nouveau introduite partiellement dans l'évaporateur via une ouverture d'entrée.

4. Procédé selon la revendication 1, **caractérisé en ce que** la partie de la méta-toluylènediamine non évaporée dans l'étape d) est évacuée de l'évaporateur par une ouverture de sortie et ensuite introduite au moins partiellement dans la distillation de toluylènediamine brute, qui est obtenue à partir de l'hydrogénation de dinitrotoluène.

5. Procédé pour la préparation de diisocyanate de méta-toluylène par transformation de méta-toluylènediamine avec du phosgène dans la phase gazeuse, dans lequel la méta-toluylènediamine et le phosgène sont introduits séparément l'un de l'autre sous forme gazeuse dans un réacteur, la méta-toluylènediamine sous forme gazeuse étant générée par évaporation dans au moins un évaporateur, **caractérisé en ce que**

a) la méta-toluylènediamine liquide introduite dans l'évaporateur contient au total moins de 0,2% en poids, par rapport au poids de méta-toluylènediamine, d'ammoniaque et d'amines cycloaliphatiques et

b) la méta-toluylènediamine liquide introduite dans l'évaporateur contient moins de 20 ppm de métaux lourds et

c) la méta-toluylènediamine introduite dans l'évaporateur est traitée thermiquement au préalable à au moins 100°C pendant au moins 15 minutes et l'ammoniaque qui se forme est séparée.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 289840 B1
- EP 593334 B1
- EP 570799 B1
- EP 1362847 B1
- EP 1449826 A1
- EP 1526129 A1
- DE 10359627 A1
- WO 2007028715 A
- WO 2008071564 A
- EP 1935876 A1
- EP 1754698 A1
- DE 2135154 B
- DE 3734344 A1
- EP 634391 B1
- DE 4435839 A1
- EP 1287884 B1
- EP 978505 B1
- EP 1033361 B1
- WO 2005066113 A1
- EP 659173 B1
- EP 794170 B1
- WO 2002048075 A1
- EP 1746083 A1
- WO 2005016512 A
- DE 102005036870 A1

**In der Beschreibung aufgeführte Nicht-Patentliteratur**

- **WILLEBOORDSE, F. ; QUICK, Q. ; BISHOP, E.T.** Direct gas chromatographic analysis of isomeric di-aminotoluenes. *Analytical Chemistry,* 1968, vol. 40 (10), 1455-1458
- **KRAUTER, J.G.E. ; GROß, M. ; PANSTER, P.** Influence of Hydrogen Supply on By-Product Formation during the Hydrogenation of DNT to TDA. *Science and Technology in Catalysis,* 2002, 427-430
- **A.R. CARTOLANO.** Kirk-Othmer Encyclopedia of Chemical Technology. John Wiley & Sons, 2001
- Ullmann. vol. 13, 351
- **A. BÜRKHOLZ.** Droplet Separation. VCH Verlagsgesellschaft, 1989